# EUROPEAN PATENT APPLICATION

(11) **EP 4 088 774 A1**
(43) Date of publication of application: **16.11.2022**
(21) Application number: 21173092.4
(22) Date of filing: 10.05.2021
(51) Int. Cl.: A61M 37/00

(54) **MICRONEEDLE BASED DELIVERY SYSTEM**

(71) Applicant: Latch Medical, 4 Dublin (IE)
(72) Inventor: BERTOLLO, Nicky, Dublin 24 (IE)
(74) Representative: FRKelly

(57) **Abstract**

The invention concerns a self-anchoring microneedle based delivery system which is operable to quickly and easily deploy microneedles into a tissue substrate such as the eye, for the purposes of targeted drug delivery and includes a body having a first section and a second section displaceable relative to one another, first hollow microneedles provided on the first section and second hollow microneedles provided on the second section, the first and second sections being displaceable relative to one another in order to transition the microneedles between a disengaged state and an engaged state.

## Description

### Field of the invention

This invention relates to a microneedle based delivery system, in particular such a system for drug or vaccine delivery to target tissue such as skin tissue with accuracy and ease of use.

### Background of the invention

Microneedles are gaining increased use in various medical applications in view of the many documented benefits for both patients and medical professionals, for example reduced tissue trauma, surgery times and patient recovery, reduced risk of infection, and minimising the surgical or medical equipment needed in procedures involving microneedle based devices. Such microneedles allow for extremely accurate, and shallow, deployment into tissue, which can be beneficial in various applications, for example in drug delivery, and more particularly in drug delivery to sensitive or delicate tissue such as skin, ocular tissue and oral mucosa.

International patent applications WO2018/069543 and WO2019/201903 provide detailed disclosures of the configuration and operation of microneedles and opposing microneedle arrays which may be provided in the form of a device for application to a tissue substrate for various surgical and therapeutic uses, one particular use being drug delivery directly from or through the microneedles provided. The disclosures of WO2018/069543 and WO2019/201903 are incorporated herein in their entirety.

Intradermal delivery of drugs and vaccines offers significant advantages over conventional intramuscular and oral delivery routes for vaccines and drugs. Systemic uptake of therapeutics via the dermal blood capillaries offers a host of benefits, including avoiding the deleterious effects of first-pass metabolism, rapid drug onset and improved bioavailability of APIs, such as biologics, that are not readily absorbed across the mucosal layers of the gastrointestinal tract. Additionally, the dermal layer of skin is replete with antigen presenting cells and therefore represents an optimal location for the delivery of vaccines in order to elicit an enhanced immune response.

The viscoelastic and highly deformable nature of skin poses significant clinical challenges in the delivery of liquid formulations to specific depths in skin using hollow microneedle and hypodermic needle-based approaches. Uncontrolled deformation of the skin significantly limits accuracy of depth targeting, which is further compounded by natural inter- and intra-subject anatomical variability in skin thickness and biomechanical properties. Skin deformation and compression caused by placement and application of these devices increases the injection pressure requirements, impacts flow-rate, and also impairs intradermal bolus diffusion kinetics. Furthermore, the absence of intrinsic anchorage of microneedle technologies, in particular, during intradermal injection requires additional mechanical work be manually applied to the patch to counter the injection pressure. Unless balanced, this component of injection pressure is driving to eject and cause relative migration of skin and/or needle tips, affecting the site of delivery but which can also result in formulation leakage, efflux and spray back. Appreciably, operator movements during handling and injection is another factor further impacting depth targeting accuracy, where the absence of intrinsic anchoring can facilitate gross movements of the needle tips relative to the skin.

The layered structure of the eye, in addition to the delicate nature of the ocular tissue and the high potential for damage or complications during any form of ocular surgery or therapy, has presented difficulties in the targeted delivery of drugs to the eye, in particular where it is required to deliver the drug to a particular layer within the eye in order to improve efficacy and potentially avoid side effects which may arise when a drug is delivered to an unintended part of the eye. Conventional drug delivery to the eye is complicated through natural and, significant variability in the thickness of the sclera (outermost layer) of the eye. In addition, the eye readily rotates during injection, requiring stabilisation, and there is deformation of the eye tissue during injection which limits the ability to target specific regions of the eye. Relative movement of the injector and drug efflux during injection also occur, further complicating the procedure and compromising the efficiency of payload delivery.

It is therefore an object of the present invention to provide a self anchoring microneedle based delivery system which is operable to quickly and easily deploy microneedles into a tissue substrate such as the skin and eye, for the purposes of targeted drug delivery.

### Summary of the invention

According to the present invention there is provided a microneedle based delivery system comprising a body having a first section and a second section displaceable relative to one another; at least one first hollow microneedle provided on the first section and at least one second hollow microneedle provided on the second section, the first and second sections being displaceable relative to one another in order to transition the microneedles between a disengaged state and an engaged state; and a delivery manifold in fluid communication with the first and second hollow microneedles.

Preferably, a longitudinal axis of the at least one first microneedle extends at a first oblique angle relative to the direction of displacement between the first and second sections, and a longitudinal axis of the at least one second microneedle extends at a second oblique angle relative to the direction of displacement between the first and second sections.

Preferably, the first oblique angle extends away from the second oblique angle.

Preferably, the at least one first microneedle is transversely offset to the at least one second microneedle relative to the direction of displacement between the first and second sections.

Preferably, the first and the second sections are slidably and/or hingedly displaceable relative to one another.

Preferably, the delivery manifold is captured between the first and second sections at least when the microneedles are in the engaged state.

Preferably, the delivery manifold is clamped against the body when the microneedles are in the engaged state such as to establish a fluid tight seal between the delivery manifold and the body.

Preferably, the delivery manifold comprises an inlet adapted for connection with a fluid reservoir and an outlet engagable with the body such that the inlet is in fluid communication with the hollow microneedles.

Preferably, the body defines a chamber between the first and second sections in which the outlet of the delivery manifold is captured and which chamber is arranged to bias the outlet into sealing engagement with the body at least when the system is in the engaged state.

Preferably, the body defines an enclosure at least partially surrounding the delivery manifold.

Preferably, the delivery manifold defines a first fluid flow path in fluid communication with the at least one first microneedle and an independent second fluid flow path in fluid communication with the at least one second microneedle.

Preferably, the first and second section of the body each define an elongate arm at a free end of which the respective at least one microneedle is provided.

Preferably, each arm defines an upper end opposite the lower end and a fluid flow path extending through the arm between the upper and lower end, the delivery manifold being in fluid communication with the fluid flow path at the upper end of each arm.

Preferably, the delivery manifold is in fluid communication with the end of the fluid flow paths when the microneedles are in both the engaged and disengaged state.

Preferably, the microneedle based delivery system comprises a release mechanism defined by a trigger on each of the first and second sections and arranged to facilitate manual displacement of the system into the disengaged state.

Preferably, the microneedle based delivery system comprises a locking mechanism to releasably secure the system in the engaged state.

Preferably, the locking mechanism is integrated with at least one of the triggers and is releasable through actuation of the trigger.

Preferably, the at least one trigger with which the locking mechanism is integrated is resiliently deformable.

Preferably, the microneedle based delivery system comprises a stop releasably engageable with the body to limit relative displacement between the first and second sections.

### Brief description of the drawings

The present invention will now be described with reference to the accompanying drawings, in which:
Figure 1 illustrates a perspective view of a microneedle based delivery system according to an embodiment of the present invention, coupled with a syringe, and in a disengaged state,
Figure 2 illustrates a front elevation of the delivery system as shown in Figure 1:
Figure 3 illustrates a front elevation of the delivery system in an engaged state;
Figure 4 illustrates a cut away perspective view of the delivery system in the disengaged state;
Figure 5 illustrates a cut away perspective view of the delivery system in the engaged state;
Figure 6 illustrates an exploded perspective view of the delivery system from a first side;
Figure 7 illustrates the exploded perspective view of Figure 6 from the reverse side;
Figure 8a illustrates a sectioned front elevation revealing a release mechanism when the system is in the engaged state;
Figure 8b illustrates the arrangement of Figure 8a with the release mechanism being activated;
Figure 8c illustrates the arrangement of Figure 8a when the system is in the disengaged state;
Figure 9 illustrates a perspective view of a microneedle based delivery system according to an alternative embodiment of the present invention and in a disengaged state;
Figure 10 illustrates a front elevation of the delivery system as shown in Figure 9;
Figure 11 illustrates a front elevation of the delivery system of Figures 9 and 10 in an engaged state;
Figure 12 illustrates a cut away view of the delivery system of Figures 9 to 11 in the engaged state;
Figure 13 illustrates a front elevation of a microneedle based delivery system according to a further alternative embodiment of the present invention and in a disengaged state;
Figure 14 illustrates a front elevation of the delivery system as shown in Figure 13 in a partially engaged state;
Figure 15 illustrates a front elevation of the delivery system of Figures 13 and 14 in a fully engaged state; and
Figure 16 illustrates a perspective view of a stop for location within the delivery system of Figures 13 to 15 to facilitate selective limitation of displacement between first and second sections of the system.

### Detailed description of the drawings

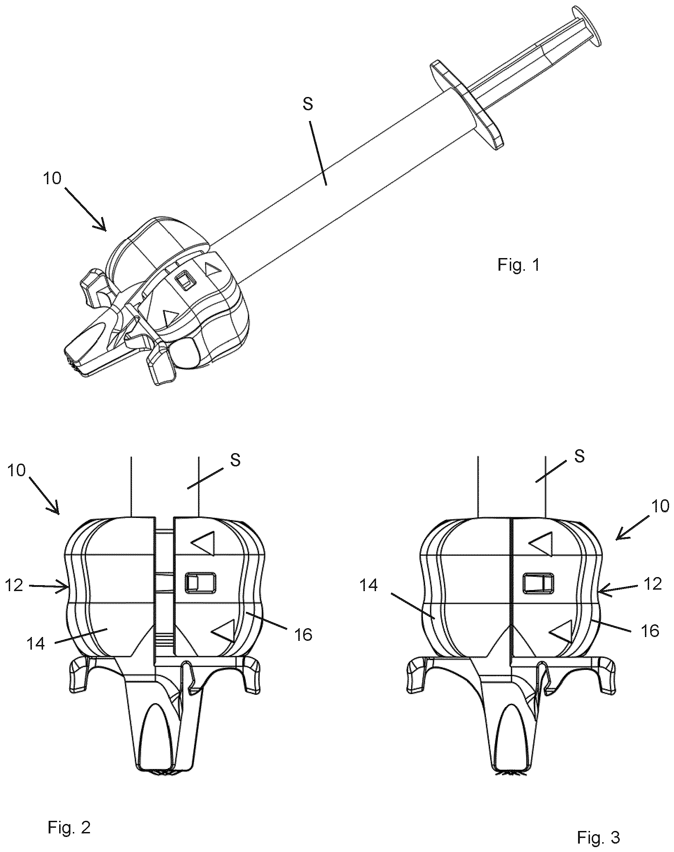

Referring now to Figures 1 to 8 of the accompanying drawings there is illustrated a microneedle based delivery system, generally indicated as 10, for use is delivering one or more fluids, in particular a dose of a drug or therapeutic agent in liquid form, to a target area of tissue. The delivery system 10 is suitable for use with a wide range of tissue, for example skin or muscle, but is particularly suited for use in delivering a drug to ocular tissue such as the subchoroidal or suprachoroidal regions of the eye (not shown). The delivery system 10 is also preferably adapted to be coupled to an external fluid supply, most preferably a conventional syringe S as will be described hereinafter, to allow a fluid to be dispensed from the syringe S or other external fluid supply to the target tissue.

The delivery system 10 comprises a body 12 having a first section 14 and a second section 16 which are respectively provided with at least one first hollow microneedle 18 and at least one second hollow microneedle 20 which are adapted to be reversibly insertable into the target tissue to allow for drug delivery through the hollow microneedle 18, 20, the operation of which will be described in detail hereinafter. The number of microneedles 18, 20 may be varied, for example to suit a particular application, target area, drug to be delivered and/or delivery rate, and it is also envisaged that one or more solid microneedles (not shown) may be provided to securely anchor the hollow microneedles 18, 20 to the target tissue to achieve reliable drug delivery. The material, dimensions, orientation, and relative positioning of the microneedles 18, 20 may also be varied as necessary. For example the dimension and/or orientation of the microneedles 18, 20 may be arranged to provide a desired depth of insertion into the target tissue, which can therefore facilitate accurate drug delivery to specific locations or layers of tissue.

In a preferred embodiment the body 12 is substantially formed from one or more polymers and the delivery system 10 is intended as a single use product. The microneedles 18, 20 may also be formed from a polymer, or may be metal or another material and suitably secured to the first and second section 14, 16. However it is also envisaged that the system 10 could be substantially formed from a metal such as stainless steel, titanium, etc. or a hard wearing polymer and may be reusable following sterilisation, for example in an autoclave. The microneedles 18, 20 could be provided as a modular component releasably securable to the body 12 and thus single use while the body 12 is reusable.

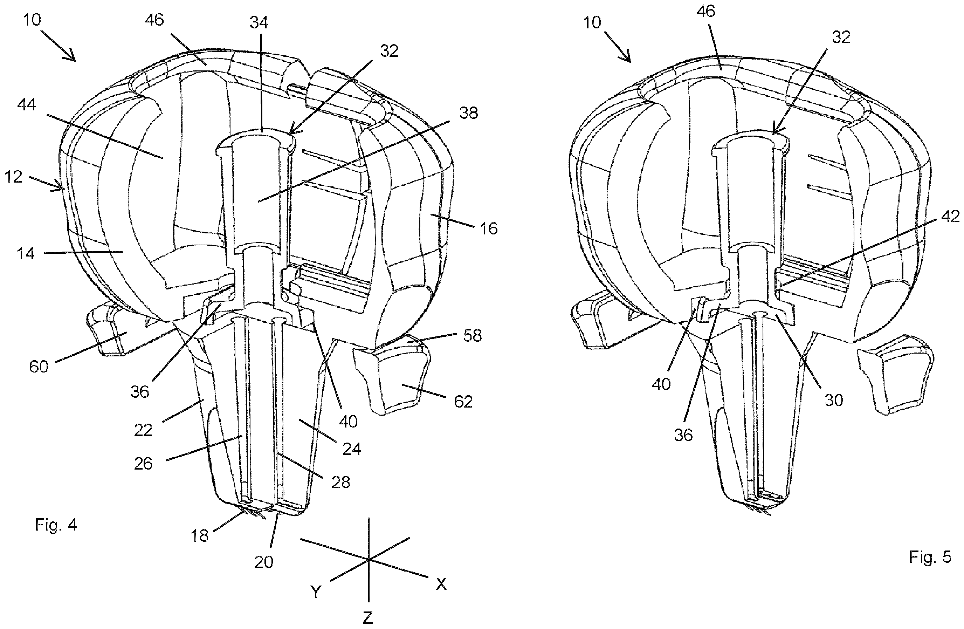

The first and second sections 14, 16 of the body 12 are secured together but displaceable relative to one another by a fixed distance, and in the embodiment illustrated are slidably displaceable relative to one another to transition the system 10, and in particular the microneedles 18, 20 between a disengaged state, for example as illustrated in Figures 1, 2 and 4, and an engaged state, for example as illustrated in Figures 3 and 5. In the disengaged stage the microneedles 18, 20 are in a first orientation relative to one another prior to application to the target tissue, and in the engaged state are in a second orientation relative to one another. In use the microneedles 18, 20 are applied against the target tissue in the disengaged state and the system 10 is then displaced into the engaged state by manually advancing the first and second section 14, 16 towards one another, which action effects the relative displacement of the microneedles 18, 20 in a manner which draws and anchors the microneedles 18, 20 into the target tissue to allow drug delivery as hereinafter described.

The underlying methodology of this deployment technique is described in detail in the above mentioned international applications WO2018/069543 and WO2019/201903. For ease of reference, hereinafter directions or dimensions in a direction between the first and second sections 14, 16 of the delivery system 10 and the microneedles 18, 20 will be referred to as being an "X" coordinate or direction, transversely or along the width will be referred to as being a 'Y" coordinate and along the depth will be referred to as being a "Z" coordinate, and as represented schematically in Figure 4 relative to the delivery system 10.

In order to allow the microneedles 18, 20 to be accurately positioned, particularly in the case of tissue that is relatively difficult to accurately engage or pierce, the body 12 comprises an elongate first arm 22 extending from the first section 14 and an adjacent elongate second arm 24 extending from the second section 16.The at least one first microneedle 18 is provided on a lower end of the first arm 22 while the at least one second microneedle 20 is provided on a lower end of the second arm 24. In the embodiment illustrated the arms 22, 24 taper inwardly as they extend away from the body 12 towards the microneedles 18, 20, thereby defining a relatively small footprint at the lower end on which the microneedles 18, 20 are located, allowing both accurate location and good visibility of the microneedles 18, 20 and surrounding tissue during application to the target tissue. It will of course be appreciated that the shape, orientation and dimensions of the arms 22, 24 may be varied as required.

In the embodiment illustrated the first and second arms 22, 24 are located adjacent one another, but offset in the "Y" direction, transverse to the "X" direction in which the first and second sections 14, 16 are displaceable relative to one another. In this way the arms 22, 24 can move into overlapping alignment as the system 10 is transitioned into the engaged state, drawing the microneedles 18, 20 into the tissue, as can be seen in particular in Figures 4 and 5. However other geometries and arrangements are also possible, for example one arm could be provided with a channel to at least partially receive the other arm when in the engaged state, or the arms may remain separated or spaced from one another in both the disengaged and engaged states.

In order to allow drug delivery through the hollow microneedles 18, 20 at least the first arm 22 is provided with a first delivery conduit 26 extending internally along the length of the arm 22 (in the "Z" direction) and in fluid communication with the at least one first microneedle 18. In the preferred embodiment illustrated the second arm 24 is provided with a second delivery conduit 28 extending internally along the length of the second arm 24 and in fluid communication with the at least one second microneedle 20. It is to be understood that the system 10 could function with a single delivery conduit to supply only the first or second microneedles 18, 20, but it is preferred that both the hollow microneedles 18, 20 can be supplied with and used for drug delivery to the target tissue.

The first and second arms 22, 24 each terminate at an upper end or face 30 onto which the delivery conduits 26, 28 open, thereby establishing a fluid flow path from the upper face 30 through the arms 22, 24 to the microneedles 18, 20. Referring to Figure 4 it can be seen that when the system 10 is in the disengaged state the upper ends of the delivery conduits 26, 28 are spaced from one another in the "X" direction, and when the system 10 is transitioned into the engaged stage as illustrated in Figure 5 the upper ends of the delivery conduits 26, 28 are adjacent one another and thus overlapping or aligned in the "X" direction. Located above and enclosing the upper ends of the deliver conduits 26, 28 is a delivery manifold 32 which comprises an inlet 34, an outlet 36 and a lumen 38 extending therebetween. The outlet 36 is in register with the upper faces 30 such as to enclose the upper ends of the delivery conduits 26, 28, thereby allowing fluid delivery through the delivery manifold 32, into the delivery conduits 26, 28, and ultimately to the microneedles 18, 20. The inlet 34 is adapted to be releasably secured to a convention luer lock connector or head H of the syringe S, but may be adapted for a fluid tight connection to any other desired external fluid supply (not shown). It will therefore be understood that the syringe S, containing a drug or other fluid to be delivered, can be connected to the system 10 via the delivery manifold 32, and fluid can thus be dispensed from the syringe S, through the microneedles 18, 20 to the target tissue in a reliable and accurate manner.

In order to secure the delivery manifold 32 to the body 12 the delivery manifold 32, and most preferably the outlet 36, is captured between the first and second sections 14, 16 at least when the microneedles 18, 20 are in the engaged state. In the embodiment of Figures 1 to 8 the body 12 defines a chamber 40 located directly above and partially defined by the upper faces 30, one part or side of the chamber 40 formed in the first section 14 and the other side of the chamber 40 being formed in the second section 16 opposite one another. The chamber 40 is shaped and dimensioned to encapsulate the outlet 36, with an opening 42 being provided in an upper wall of the chamber 40 through which the delivery manifold 32 extends into an enclosure 44 defined by the body 12. The enclosure 44 is preferably shaped and dimensioned to surround and effectively enclose the delivery manifold 32 such that, in use, the delivery manifold 32, and therefore the connection to the syringe S, is inaccessible. A mouth 46 is formed in an upper region of the enclosure 44 to accommodate the syringe S. As the mouth 46 extends across the interface between the first and second sections 14, 16 it will be appreciated that with the system 10 in the disengaged state the mouth 46 will be enlarged to allow the syringe S to be advanced into the chamber 44 to couple the head H with the inlet 34 of the delivery manifold 32. When the system 10 is transitioned into the engaged state the mouth 46 will partially close about the body of the syringe S to avoid any unintentional movement or uncoupling of the syringe S.

At the interface between the outlet 36 of the delivery manifold 32 and the upper face 30 of the arms 22, 24, as seen in particular in Figures 4 and 5, the outlet 36 is dimensioned to enclose the upper ends of the delivery conduits 26, 28 when the system 10 is in both the disengaged state of Figure 4 and the engaged state of Figure 5, although the system 10 could be adapted such that the outlet 36 only encloses the upper ends of the delivery conduits 26, 28 when the system 10 is in the engaged state. However, it is often necessary to prime a syringe prior to dispensing a fluid, in order to ensure there is no air in the syringe that would then be injected into the subject. Thus by having the outlet 36 dimensioned to enclose the upper ends of the delivery conduits 26, 28 when the system 10 is in the disengaged state the syringe S can be primed or purged of air before application of the microneedles 18, 20 to the target tissue, as a fluid flow path exists between the syringe S and the microneedles 18, 20. When the microneedles 18, 20 are secured to the target tissue the system 10 will be in the engaged state, and as the outlet 36 also encloses the upper ends of the delivery conduits 26, 28 in this state the contents of the syringe S can then be delivered to the target tissue via the microneedles 18, 20.

It is envisaged that the manifold 32 could be replaced with a modified manifold (not shown) defining a pair of independent fluid flow paths each arranged in fluid communication with one of the delivery conduits 26, 28, thereby allowing two different fluids to be delivered to the first and second microneedles 18, 20 via a modified syringe (not shown) or pair of syringes (not shown) or other fluid supplies.

In order to prevent unwanted leakage of the contents of the syringe S at the interface between the outlet 36 and the upper face 30 of the arm 22, 24 a suitable fluid tight seal is established at the interface. This seal may be achieved in a number of ways, for example using gaskets, coatings, sealing geometries, mechanical interlocking or an interference fit between the outlet 36 and the upper face 30 of the arms 22, 24. In the embodiment of Figures 1 to 8 this seal is established by complimentary dimensioning of the outlet 36 and the chamber 40, in particular in the "Z" direction whereby the height or "Z" dimension of the chamber 40 is such that the upper wall of the chamber 40 contacts the upper wall of the outlet 36 and biases the outlet 36 against the upper face 30 of the arms 22, 24 such as to establish a seal therebetween. In a particularly preferred arrangement, the upper wall of the chamber 40 and the upper wall of the outlet 36, which are in face to face engagement, have a corresponding draft or incline, for example in the region of 1 ° or 2° in the "X" direction of relative displacement between the first and second sections 14, 16. In this way, as the first and second sections 14, 16 are displaced towards one another in transitioning the system 10 into the engaged state, the bias applied to the outlet 36 will increase, thereby increasing the seal at the interface between the outlet 36 and the upper face 30 of the arms 22, 24. This arrangement increases the mechanical compressive force acting on the outlet 36 from nominal in the disengaged state to maximal in the engaged state. This allows for the above described priming of the syringe S and microneedles 18, 20 prior to injection into tissue, where pressures are low, to allowing higher pressures associated with the injection once the microneedles 18, 20 have been deployed into the tissue, without fear of leakage.

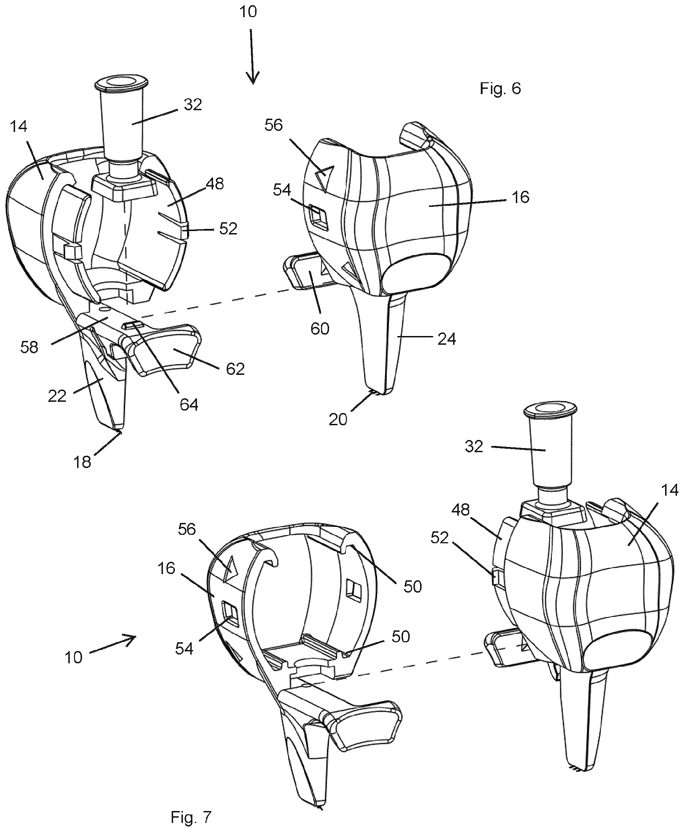

In use the delivery system 10 is transitioned from the disengaged state to the engaged state by manually applying pressure to the first and second sections 14, 16 of the body 12, preferably via the outer walls of the enclosure 44. As seen in Figures 6 and 7 the walls of body 12 which define the enclosure 44 include extensions 48 on the first section 14 and corresponding guideways 50 on the second section 16 which interlock to secure the sections 14, 16 together while permitting limited relative displacement. Tabs 52 are provided in the extensions 48 which will be visible through corresponding windows 54 when the system 10 has been correctly transitioned into the engaged state to provide a visual indication to the user signifying that the microneedles 18, 20 are embedded and fluid delivery from the syringe S can be undertaken. The tabs 52 further serve to ensure that the first and second sections 14, 16 can not disengage from one another. Graphical indicia 56 may be provided about the body 12 to indicate the correct direction in which the first and second sections 14, 16 should be displaced to transition the system 10 into the engaged state. The system 10 may be provided with a stop (not shown) releasably engageable with the body 12, for example located within the enclosure 44, to limit relative displacement between the first and section sections 14, 16 as a means of limiting the depth of penetration of the microneedles 18, 20 to suit a particular application or anatomical consideration.

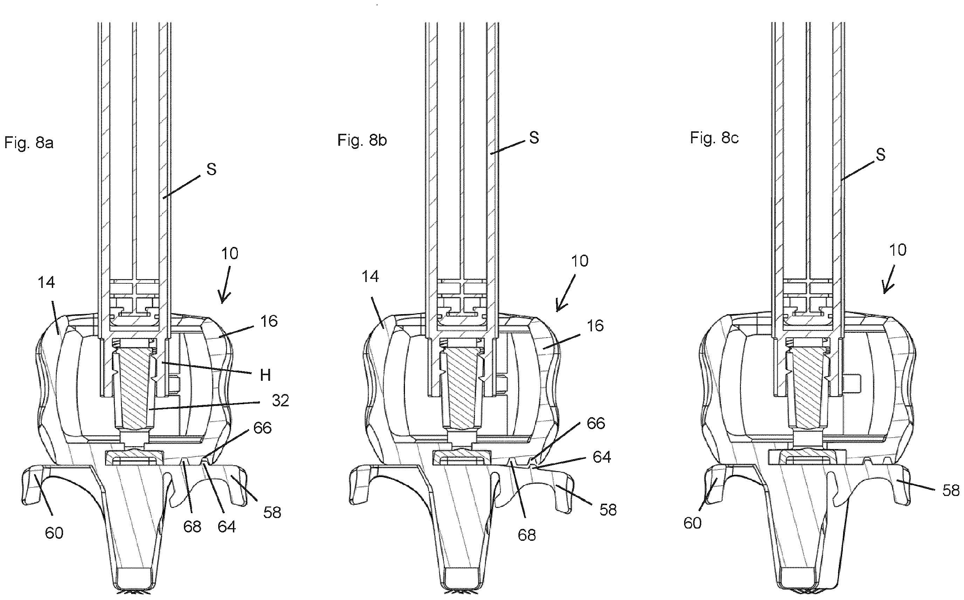

Once delivery of fluid to the target tissue has been completed it is necessary to disengage the system 10, in particular the microneedles 18, 20, from the tissue. It is therefore necessary for the user to displace the first and second sections 14, 16 away from one another to transition the system 10 into the disengaged state and thus affect withdrawal of the microneedles 18, 20. In order to facilitate this action the system 10 comprises a release mechanism in the form of first and second triggers 58, 60 respectively provided on the first and second sections 14, 16, in the region of the upper face 30 of the arms 22, 24 and positioned beneath the enclosure 44. Each trigger 58, 60 extends in cantilever form towards and marginally beyond the wall of the enclosure 44 of the opposite section 14, 16 in order to be accessible by the user. Each trigger 58, 60 terminates in an enlarged paddle 62 which is shaped and dimensioned for operative engagement with a finger or thumb of the user. The triggers 58, 60 are therefore positioned side by side and slide relative to one another when the system 10 is displaced between the disengaged and engaged states without hindering said displacement of the system 10. Referring to Figure 6 the system 10 is preferably provided with a locking mechanism to secure the system 10 in the engage and or disengaged state, in particular to avoid any inadvertent withdrawal of the microneedles 18, 20, for example while fluid is being delivered to the tissue. In the embodiment illustrated the locking mechanism is provided in the form of a protrusion or detent 64 on an upper face of the first trigger 58 and a correspondingly shaped and dimensioned first socket 66 and second socket 68 on the underside of the enclosure 44 which is in face to face with the upper face of the first trigger 58, and visible in Figures 8a, 8b and 8c. The first and second sockets 66, 68 are spaced apart from one another by the distance through which the first and second sections 14, 16 are displaceable. The first socket 66 is located to receive the detent 64 when the system 10 is in the engaged state as illustrated in Figure 8a, to releasably secure the system 10 in the engaged state.

Referring in particular to Figures 8a, 8b and 8c the sequence of steps to effect displacement of the system 10 from the engaged to disengaged state is illustrated. Figure 8a shows the system 10 in the engaged state with the detent 64 captured in the first socket 66. In order to begin displacing the first and second sections 14, 16 away from one another the system 10 is gripped by the triggers 58, 60, for example between thumb and forefinger. At least the first trigger 58 is resiliently deformable, and the user therefore applies pressure to the first trigger 58 via the paddle 62 to deform the first trigger 58 in a manner which will draw the detent 64 out of the first socket 66 as illustrated in Figure 8b. At this point the user can apply pressure to both triggers 58, 60 to effectively push the triggers 58, 60 towards one another. This will effect relative displacement between the first and second sections 14, 16 to transition the system 10 into the disengaged state as shown in Figure 8c, drawing the microneedles 18, 20 out of the tissue. In this configuration the detent 64 will be aligned with the second socket 68 and once pressure is released the first trigger 58 will return and the detent 64 will enter the second socket 68 to lock the system 10 in the disengaged state. The system 10 can then be withdrawn from the tissue.

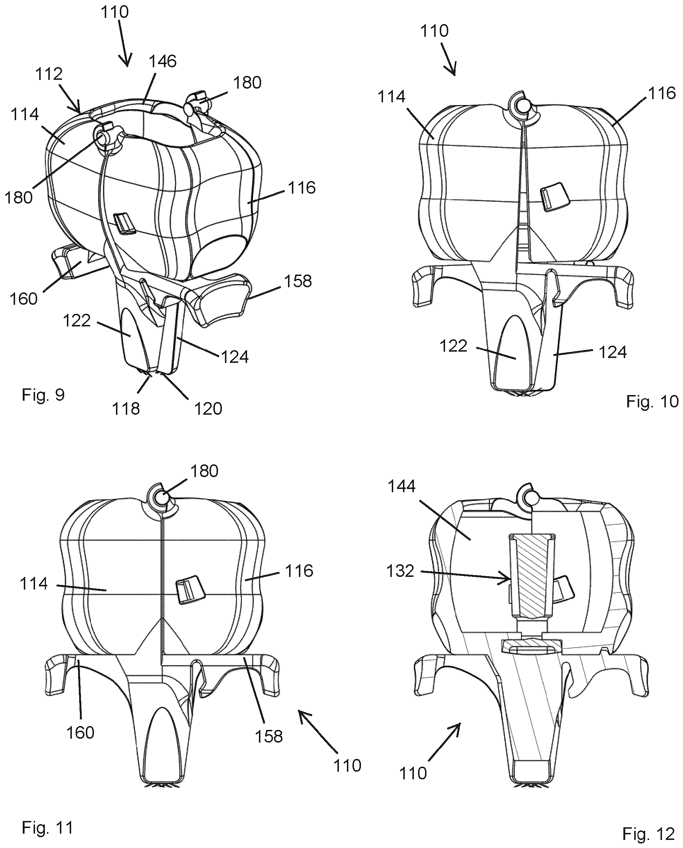

Referring now to Figures 9 to 12 there is illustrated an alternative embodiment of a microneedle based delivery system according to the present invention, generally indicated as 110. In this alternative embodiment like components have been accorded like reference numerals and unless otherwise stated perform a like function.

The delivery system 110 comprises a body 112 having a first section 114 and a second section 116 which are displaceable relative to one another to transition the system 110 between disengaged and engaged states. The system 110 comprises first hollow microneedles 118 provided on the first section 114 and second hollow microneedles 120 provided on the second section 116. The body 112 defines elongate first and second arms 122, 124 at a free end of which the microneedles 118, 120 are provided. Although not illustrated, first and second delivery conduits (not shown) extend through the arms 122, 124 into fluid communication with the microneedles 118, 120. A delivery manifold 132 is captured between the first and second sections 114, 116 to facilitate the coupling of a syringe (not shown) or other fluid supply to the system 110 for delivery through the microneedles 118, 120 in the same manner as described above in relation to the system 10 shown in Figures 1 to 8. The body 112 defines an enclosure144 above the arms 122, 124 in which the delivery manifold 132 is contained and which is accessible through a mouth 146 at an upper portion of the enclosure 144.

The overall composition, appearance and general operation of the system 110 is the same as described above with reference to the system 10 shown in Figures 1 to 8. However, unlike the system 10 the first and second sections 114, 116 of the system 110 are pivotally displaceable relative to one another in order to transition the system 110 between the disengaged and engaged states. The first and second sections 114, 116 are secured to one another by means of a pair of hinges 180 which are located on opposite sides of the mouth 146, although it will be appreciated that numerous other configurations may be employed to enable this pivoting relative displacement.

Thus as the first and second sections 114, 116 are displaced towards the engaged state the microneedles 118, 120 will move relative to one another along an arc, advancing the microneedles 118, 120 into the target tissue. Once the system 110 is in the engaged stage with the microneedles 118, 120 embedded in the tissue a fluid can be delivered into the tissue from the coupled syringe (not shown) in the same manner as described above for the system 10. The system 110 may be provided with a stop (not shown) releasably engageable with the body 112, for example located within the enclose 144, to limit relative displacement between the first and section sections 114, 116 as a means of limiting the depth of penetration of the microneedles 118, 120.

Similarly the system 110 may be disengaged from the tissue following fluid delivery by utilising a release mechanism in the form of a pair of triggers 158, 160 to displace the first and second sections 114, 116 away from one another along an arcuate path about the pair of hinges 180 in a similar manner as described above.

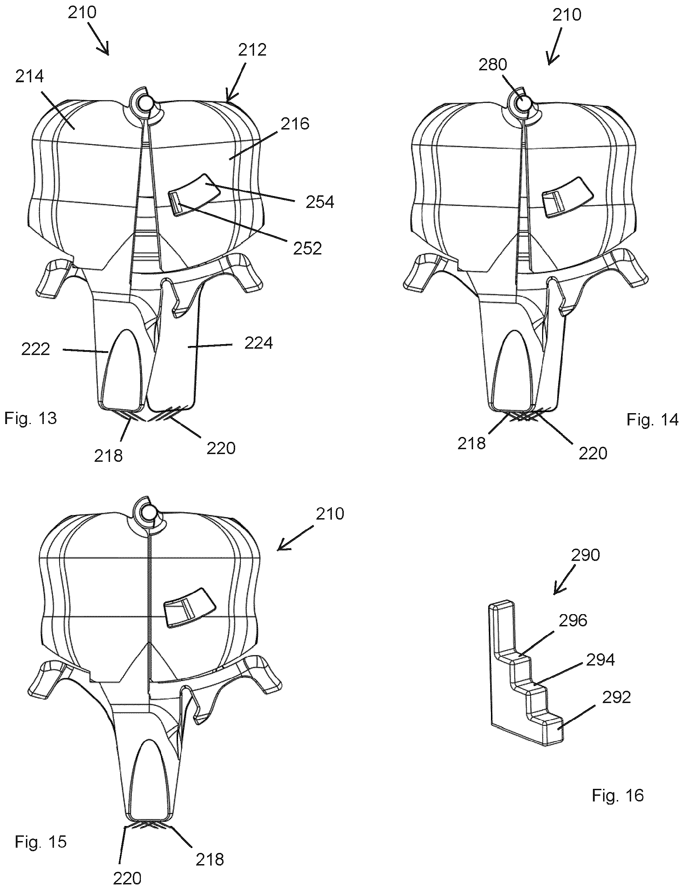

Referring to Figures 13 to 16 there is illustrated a further alternative embodiment of a microneedle based delivery system according to the present invention, generally indicated as 210. In this alternative embodiment like components have been accorded like reference numerals and unless otherwise stated perform a like function.

The delivery system 210 is effectively a variant of the system 110, comprising a body 212 having a first section 214 and a second section 216 which are again hingedly displaceable relative to one another to transition the system 210 between disengaged and engaged states. The system 210 comprises first hollow microneedles 218 provided on the first section 214 and second hollow microneedles 220 provided on the second section 216. In this embodiment the microneedles 218, 220 are of increased length relative to the previous embodiments in order to permit increased tissue penetration.

The body 212 defines elongate first and second arms 222, 224 at a free end of which the microneedles 218, 220 are provided. The first and second sections 214, 216 of the system 210 are secured to one another by means of a pair of hinges 280, although it will again be appreciated that numerous other configurations may be employed to enable this pivoting relative displacement. Tabs 252 provided on the first section 214 are located within windows 254 on the second section 216 to both provide a visual indication as to the level of displacement of the first and second sections 214, 216 and thus deployment of the microneedles 218, 220, and to prevent the first and second sections 214, 216 from disengaging from one another.

The system 210 is optionally provided with a stop 290 shown in isolation in Figure 16, and which in use is located within a chamber (not shown) defined between the first and second sections 214, 216 of the body 212 and arranged to selectively limit relative displacement between the first and second sections 214, 216 as a means of limiting the depth of penetration of the microneedles 218, 220. The stop 290 comprises first, second and third steps 292, 294, 296 of increasing height, whereby the stop 290 may be selectively positioned such that one of the steps 292, 294, 296 projects outwardly though the window 254 such as to be captured between the tab 252 and the window 254. The depth of the step 292, 294, 296 located in the window 254 will determine to what extent the first and second sections 214, 216 may be displaced together and thus to what extent the system 210 can be displaced into the engaged state. For example Figure 14 shows the system 210 displaced into a partially engaged state with the microneedles 218, 220 in a first relative position while Figure 15 shows the system 210 displaced into a fully engaged state with the microneedles 218, 220 in a second relative position which will achieve a greater tissue penetration than that of Figure 14. These two states may be permitted by locating different steps 292, 294, 296 of the stop 290 within the window 254. The stop 290 may also be positioned with the largest step 296 projecting through the window 254 which may be dimensioned to effectively lock the system 210 in the disengaged state to prevent accidental deployment of the microneedles 218, 220, for example during handling, particularly when applying a torque to a syringe (not shown) being coupled to the system 210.

The microneedle based delivery system 10, 110; 210 of the present invention thus provides a means of effecting the highly targeted delivery of a fluid such as a drug to a target site, allowing accurate surface placement and depth selection, while being simple to operate and permitting connection to a conventional syringe, and having particular utility in ocular treatment by facilitating ease of engagement and anchoring while also allowing the accurate delivery of a drug or vaccine to a desired depth in the skin or layer of the eye such as the subchoroidal or suprachoroidal regions.

The invention is not limited to the embodiments described herein but can be amended or modified without departing from the scope of the present invention.

## Claims

1. A microneedle based delivery system comprising a body having a first section and a second section displaceable relative to one another; at least one first hollow microneedle provided on the first section and at least one second hollow microneedle provided on the second section, the first and second sections being displaceable relative to one another in order to transition the microneedles between a disengaged state and an engaged state; and a delivery manifold in fluid communication with the first and second hollow microneedles.

2. The microneedle based delivery system of claim 1 in which a longitudinal axis of the at least one first microneedle extends at a first oblique angle relative to the direction of displacement between the first and second sections, and a longitudinal axis of the at least one second microneedle extends at a second oblique angle relative to the direction of displacement between the first and second sections.

3. The microneedle based delivery system of claim 2 in which the first oblique angle extends away from the second oblique angle.

4. The microneedle based delivery system of claim 2 or 3 in which the at least one first microneedle is transversely offset to the at least one second microneedle relative to the direction of displacement between the first and second sections.

5. The microneedle based delivery system of any preceding claim in which the first and the second sections are slidably and/or hingedly displaceable relative to one another.

6. The microneedle based delivery system of any preceding claim in which the delivery manifold is captured between the first and second sections at least when the microneedles are in the engaged state.

7. The microneedle based delivery system of claim 6 in which the delivery manifold is clamped against the body when the microneedles are in the engaged state such as to establish a fluid tight seal between the delivery manifold and the body.

8. The microneedle based delivery system of any preceding claim in which the delivery manifold comprises an inlet adapted for connection with a fluid reservoir and an outlet engagable with the body such that the inlet is in fluid communication with the hollow microneedles.

9. The microneedle based delivery system of claim 8 in which the body defines a chamber between the first and second sections in which the outlet of the delivery manifold is captured and which chamber is arranged to bias the outlet into sealing engagement with the body at least when the system is in the engaged state.

10. The microneedle based delivery system of any preceding claim in which the body defines an enclosure at least partially surrounding the delivery manifold.

11. The microneedle based delivery system of any preceding claim in which the first and second section of the body each define an elongate arm at a free end of which the respective at least one microneedle is provided.

12. The microneedle based delivery system of any preceding claim in which each arm defines an upper end opposite the lower end, and a fluid flow path extending through the arm between the upper and lower end, the delivery manifold being in fluid communication with the fluid flow path at the upper end of each arm.

13. The microneedle based delivery system of claim 12 in which the delivery manifold is in fluid communication with the end of the fluid flow paths when the microneedles are in both the engaged and disengaged state.

14. The microneedle based delivery system of any preceding claim comprising a release mechanism defined by a trigger on each of the first and second sections and arranged to facilitate manual displacement of the system into the disengaged state.

15. The microneedle based delivery system of any preceding claim comprising a stop releasably engageable with the body to limit relative displacement between the first and section sections.
